# EUROPEAN PATENT SPECIFICATION

(11) **EP 0 977 583 B1**
(45) Date of publication and mention of the grant of the patent: **04.09.2002**
(21) Application number: 98918399.1
(22) Date of filing: 16.04.1998
(51) Int. Cl.: A61K 38/22

(54) **COMPOSITIONS COMPRISING CONJUGATES OF STABLE, ACTIVE, HUMAN OB PROTEIN WITH IMMUNOGLOBULIN FC CHAIN AND METHODS**
ZUSAMMENSETZUNGEN AUS KONJUGATEN DES STABILEN, AKTIVEN, MENSCHLICHEN OB PROTEINS MIT DER FC KETTE VON IMMUNOGLOBULINEN UND DAMIT ZUSAMMENHÄNGENDE VERFAHREN
COMPOSITIONS COMPRENANT DES CONJUGUES DE PROTEINE OB HUMAINE, ACTIVE, STABLE AVEC UNE CHAINE FC D'IMMUNOGLOBULINS ET LEURS PROCEDES

(30) Priority: 17.04.1997 US 843971; 14.04.1998 US 59467
(43) Date of publication of application: 09.02.2000
(73) Proprietor: Amgen Inc., Thousand Oaks, CA 91320-1799 (US)
(72) Inventor: BREMS, David, N., Newbury Park, CA 91320 (US); FRENCH, Donna, L., Moorpark, CA 93021 (US); SPEED, Margaret, A., Newbury Park, CA 91320 (US)
(74) Representative: Richardson, Kate
(86) International application number: US9807828
(87) International publication number: WO98046257

(56) References cited:
- WO-A-96/23518
- WO-A-97/00319
- WO-A-97/24440
- WO-A-98/28427

## Description

### Field of the Invention

The present invention relates to stable, active human OB protein compositions at high concentrations and at or near physiologic pH. Also disclosed are related compositions, methods of manufacture and methods of using such compositions.

### Background

Although the molecular basis for obesity is largely unknown, the identification of the "OB gene" and protein encoded ("OB protein") has shed some light on mechanisms the body uses to regulate body fat deposition. Zhang et al., Nature 372: 425-432 (1994); see also, the Correction at Nature 374: 479 (1995). PCT publication No. WO 9.6/05309, published February 22, 1996, entitled, "Modulators of Body Weight, Corresponding Nucleic Acids and Proteins, and Diagnostic and Therapeutic Uses Thereof" fully sets forth OB protein and related compositions and methods. An amino acid sequence for human OB protein is set forth at WO 96/05309 SEQ ID NOS. 4 and 6 (at pages 172 and 174 of that publication), and the first amino acid residue of the mature protein is at position 22 and is a valine residue. The mature protein is 146 residues (or 145 if the glutamine at position 49 is absent, SEQ ID NO. 6).

The OB protein is active in vivo in both *ob*/*ob* mutant mice (mice obese due to a defect in the production of the OB gene product) as well as in normal, wild type mice. The biological activity manifests itself in, among other things, weight loss. See generally, Barinaga, "Obese" Protein Slims Mice, Science 269: 475-476 (1995) and Friedman, "The Alphabet of Weight Control," Nature 385: 119-120 (1997). It is known, for instance, that in *ob*/*ob* mutant mice, administration of OB protein results in a decrease in serum insulin levels, and serum glucose levels. It is also known that administration of OB protein results in a decrease in body fat. This was observed in both *ob*/*ob* mutant mice, as well as non-obese normal mice. Pelleymounter et al., Science 269: 540-543 (1995); Halaas et al., Science 269: 543-546 (1995). See also, Campfield et al., Science 269: 546-549 (1995) (Peripheral and central administration of microgram doses of OB protein reduced food intake and body weight of *ob*/*ob* and diet-induced obese mice but not in db/db obese mice.) In none of these reports have toxicities been observed, even at the highest doses.

For preparation of a pharmaceutical composition for injection in humans, it has been observed that the human amino acid sequence is insoluble at physiologic pH at relatively high concentrations, such as above about 2 mg active protein/milliliter of liquid. Dosages in the milligram protein per kilogram body weight range, such as .5 or 1.0 mg/kg/day or below, are desirable for injection of therapeutically effective amounts into larger mammals, such as humans. An increase in protein concentration is necessary to avoid injection of large volumes, which can be uncomfortable or possibly painful to the patient.

With the advances in recombinant DNA technology, the availability of recombinant proteins for therapeutic use has engendered advances in protein formulation. A review article describing protein modification and fusion proteins is Francis, *Focus on Growth Factors* 3:4-10 (1992).

One such modification is the use of the Fc region of immunoglobulins. Antibodies comprise two functionally independent parts, a variable domain known as "Fab", which binds antigen, and a constant domain, known as "Fc" which provides the link to effector functions such as complement or phagocytic cells. The Fc portion of an immunoglobulin has a long plasma half-life, whereas the Fab is short-lived. Capon *et al.,* Nature 337: 525-531 (1989).

Therapeutic proteins have been constructed using the Fc domain to provide longer half-life or to incorporate functions such as Fc receptor binding, protein A binding, complement fixation and placental transfer which all reside in the Fc proteins of immunoglobulins. *Id.* For example, the Fc region of an IgG1 antibody has been fused to the N-terminal end of CD30-L, a molecule which binds CD30 receptors expressed on Hodgkin's Disease tumor cells, anaplastic lymphoma cells, T-cell leukemia cells and other malignant cell types. See, U.S. Patent No. 5,480,981. IL-10, an antiinflammatory and anti-rejection agent has been fused to murine Fcγ2a in order to increase the cytokine's short circulating half-life. Zheng, X. *et al.,* The Journal of Immunology, 154: 5590-5600 (1995). Studies have also evaluated the use of tumor necrosis factor receptor linked with the Fc protein of human IgG1 to treat patients with septic shock. Fisher, C. *et al.,* N. Engl. J. Med., 334: 1697-1702 (1996); Van Zee, K. *et al*., The Journal of Immunology, 156: 2221-2230 (1996). Fc has also been fused with CD4 receptor to produce a therapeutic protein for treatment of AIDS. See, Capon *et al*., Nature, 337:525-531 (1989). In addition, the N-terminus of interleukin 2 has also been fused to the Fc portion of IgG1 or IgG3 to overcome the short half life of interleukin 2 and its systemic toxicity. See, Harvill *et al*., Immunotechnology, 1: 95-105 (1995).

For insulin, suspension preparations have been reported. But, those conditions which are applicable to insulin are not predictive of those conditions which may be applicable to any other protein, including OB protein. Insulin is a fairly small protein, having particular physical and chemical characteristics; these characteristics are important to determining conditions for formulation. Brange Galenics of Insulin, Springer-Verlag 1987 describes insulin suspensions (p.36); see also, Schlichtkrull et al. Insulin Preparations with Prolonged Effect, pp. 729-777 In: Hassellblatt et al., Handbook of Experimental Pharmacology New Series, Vol. XXXII-1/2 Springer-Verlag Berlin, Heidelburg, New York (1975).

A frozen or lyophilized form of human OB protein may be used to improve shelf-stability but, are less desirable than the ready-to-use suspension forms described here. A frozen form requires storage at a constant frozen temperature which may not be possible due to defrost cycles of consumer-grade refrigerators and freezers. Furthermore, a lyophilized form must be diluted and mixed which is inconvenient and may compromise patient compliance. From a producer's point of view, manufacturing, storing and shipping of frozen liquid is expensive and requires far more supervision than distribution of a ready to use formulation. Also, manufacture or otherwise making available a suitable diluent for a lyophilized formulation is more costly and less efficient than not requiring such diluent. There exists a need for concentrated forms of human pharmaceutical compositions containing active OB protein which can be delivered by injection at low volumes. The present invention fulfills these requirements.

### Summary of the Invention

The present invention stems from the observation that certain suspension formulations allow the preparation of stable human Fc-OB protein in concentrations of at least 0.5 mg/ml at physiological pH. As used herein, the term, "physiological pH" refers to a pH of 6.0 to 8.0. Use of such compositions allow for delivery of relatively low volumes of OB protein therapeutic. As further disclosed herein, the use of precipitating agents allows for the preparation of suspensions of human OB protein having the characteristics of:
1. Improved stability at physiological pH as compared to the same human OB protein in solution form. Demonstrated below in the working examples are human OB protein suspensions at concentrations of 10 mg/ml or higher and at pH 7, which, when compared to the same concentration in solution at the highest pH which allows solubility (pH 4 for human OB protein in its native form, which is not even physiologic pH) have better HPLC (high pressure liquid chromatography) profiles. Also demonstrated below is a Fc-OB fusion protein suspension at a concentration of 5 mg/ml at about pH 7 which shows less degradation products upon storage than a comparable Fc-OB fusion protein solution.
2. Sustained infection time-release profile as compared to the same human OB protein in solution form. The working examples below demonstrate a sustained-release effect using highly concentrated human OB protein suspensions. Such sustained release effect is advantageous in that the activity of material is maintained over a relatively longer period of time, and thus there is a relatively higher potency, and the need for fewer injections as compared with human OB protein in solution.

In one example, a stable preparation of active human OB protein at a concentration of at least 2.0 mg/ml and a pH of 7.0 is disclosed. The upper limit of concentration is that suspension form which is available for administration to a human, and, as described below, working examples have provided a concentration of as high as 100 mg/ml at physiologic pH (e.g., between pH 6.0 and pH 8.0).

In another example, a stable preparation of active human OB protein within the pH range of 6.0 to 8.0 having a concentration of at least about 10 mg/ml is disclosed.

Also disclosed are formulations for stable, active human OB protein at a concentration of 2 mg protein/ml or above, at pH 6.5 to pH 7.5. More particularly, formulations for stable, active human OB protein at a concentration of 20 mg/ml to 100 mg/ml at pH 7.0 are disclosed.

Further disclosed are formulations for stable, active human OB protein at a concentration of 10 mg/ml or above at a pH of 5.0 to 8.0

Also disclosed are pharmaceutical compositions of the above, methods for manufacturing such compositions, methods of treatment using present compositions, and methods of manufacturing medicaments containing the present compositions for such treatment.

The present invention relates to formulations for stable, active human OB protein derivatized by the attachment of an Fc region of an immunoglobulin at a concentration of 0.5 mg/ml or above and a pH of 6.0 to 8.0. More particularly, formulations for stable, active Fc-OB fusion protein at a concentration of 5 mg/ml to 50 mg/ml at about pH 7.5 are disclosed.

According to the present invention, there is provided a suspension having a pH of 6.0 to 8.0 of human OB protein derivatized by the attachment of an Fc region of an immunoglobulin to the N-terminus of the OB protein moiety at a concentration of at least 0.5mg/ml, said suspension also containing a precipitating agent.

Preferably, the Fc portion of said human Fc-OB protein is selected from the group consisting of:
(a) the Fc amino acid sequences as set forth in SEQ ID NOS: 2 and 4;
(b) the amino acid sequence of subpart (a) having a different amino acid substituted or deleted in one or more of the following positions (using the numbering according to SEQ ID NO: 2):
   (i) one or more cysteine residues replaced by an alanine or serine residue;
   (ii) one or more tyrosine residues replaced by a phenylalanine residue;
   (iii) the amino acid at position 5 replaced with an alanine;
   (iv) the amino acid at position 20 replaced with glutamic acid;
   (v) the amino acid at position 103 replaced with an alanine;
   (vi) the amino acid at position 105 replaced with an alanine;
   (vii) the amino acid at position 107 replaced with an alanine;
   (viii) the amino acids at positions 1, 2, 3, 4 and 5 deleted;
   (ix) one or more residues replaced or deleted to ablate the Fc receptor binding site;
   (x) one or more residues substituted or deleted to ablate the complement (C1q) binding site; and
   (xi) a combination of subparts i-x; and
(c) the amino acid sequence of subparts (a) or (b) having a methionyl residue at the N-terminus.

Also provided is a method of preparing a suspension of the present invention, a suspension of the invention for therapeutic administration, and the use of a suspension of the present invention for the manufacture of a medicament for treatment of a condition selected from weight modulation, adiposity modulation, diabetes, blood lipid level modulation, increase in lean mass and increase in insulin sensitivity. Further provided is a pharmaceutical composition comprising a suspension of the present invention, and a method of cosmetically treating an individual comprising administering to said individual an effective dose of a suspension of the present invention.

### Brief Description of the Drawings

FIGURES 1A and 1B are dose response curves in mice for (1A) the human OB suspension of Example 1 and (1B) a control human OB protein solution as described in Example 1.

FIGURE 2 is a graph illustrating OB serum levels in dogs for the OB protein suspension of Example 2 and for a control human OB protein solution as described in Example 1.

FIGURE 3 is a reversed phase high pressure liquid chromatography (RP-HPLC) tracing of human OB protein formulations at 37°C for seven weeks: the human OB protein zinc suspension of Example 1 is the middle tracing, the human OB protein solution control of Example 1 is the top line and a human OB protein suspension kept at -80°C for 56 days is the bottom line.

FIGURE 4 is a RP-HPLC tracing of human OB protein formulations at 19°C for 56 days: human OB solution of Example 1 is the top line, the human OB crystalline suspension of Example 2 is the middle line, and the human OB crystalline suspension of Example 2 at -80°C for 56 days is the bottom line.

FIGURES 5A-5C are the DMA sequence (SEQ ID NO: 1) and amino acid sequence (SEQ ID NO: 2) of a human metFc-OB protein.

FIGURES 6A-6C are the DNA sequence (SEQ ID NO: 3) and amino acid sequence (SEQ ID NO: 4) of a human metFc-OB protein variant.

FIGURE 7 is a graph depicting the rate of formation of iso asp at asp 108 (asp 335 using the numbering according to SEQ ID NO: 4), as determined by reverse phase HPLC, for recombinant methionyl human Fc-OB protein.

### Detailed Description

The stable, active OB protein compositions disclosed herein are generally classified as suspensions, in that the protein moiety is precipitated and suspended in a liquid moiety. The compositions contain an active OB protein moiety, a precipitating agent, a pH modulating agent, and a liquid carrier. The OB proteins may be either amorphous or in crystalline form.

Preferably, for use as a therapeutic or cosmetic composition in humans, OB protein with the amino acid sequence of native human OR protein (see Zhang ek al., Nature, supra), optionally with an N-terminal methionyl residue incident to bacterial expression, is used. See, PCT Publication WO 96/05309, for recombinant DNA means to prepare OB proteins which may be used in the suspensions of the present invention. One may make changes in selected amino acids so long as such changes preserve the overall folding or activity of the protein. Table 1, below sets forth conserved amino acid substitutions which may be used, in terms of particular characteristics (basic, acidic, polar, hydrophobic, aromatic, and size (small)). See, in general, Ford et al., Protein Expression and Purification 2: 95-107, 1991. Small amino terminal extensions, such as an amino-terminal methionine residue, a small linker peptide of up to about 20-25 residues, or a small extension that facilitates purification, such as a poly-histidine tract, an antigenic epitope or a binding domain, may also be present.

**Table 1**

| Conservative Amino Acid Substitutions | |
|---|---|
| Basics | arginine |
| | lysine |
| | histidine |
| Acidic | glutamic acid |
| | aspartic acid. |
| Polar | glutamine |
| | asparagine |
| Hydrophobic | leucine |
| | isoleucine |
| | valine |
| Aromatic | phenylalanine |
| | tryptophan |
| | tyrosine |
| Small | glycine |
| | alanine |
| | serine |
| | threonine |
| | methionine |

Generally, human OB proteins which will display increased stability in the suspensions of the present invention will be those which, upon exposure to physiologic pH, have an exposed hydrophobic region when in solution.

Human OB proteins derivatized by the attachment of an Fc region of an immunoglobulin to the N-terminus of the OB protein moiety display increased stability in the suspensions of the present invention.

Generally, an Fc region of an immunoglobulin may be genetically or chemically fused to a human OB protein. In the suspensions of the present invention, the Fc region is fused at the N-terminus of the OB protein. See copending U.S. application Serial No. 08/770,973, filed December 20, 1996, for preferred Pc-OB fusion proteins.

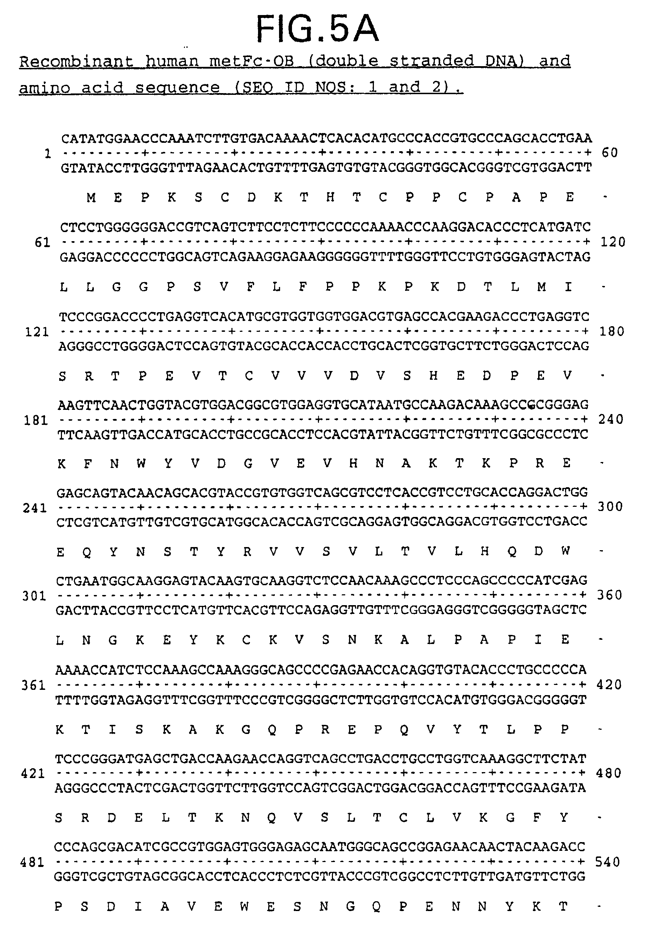

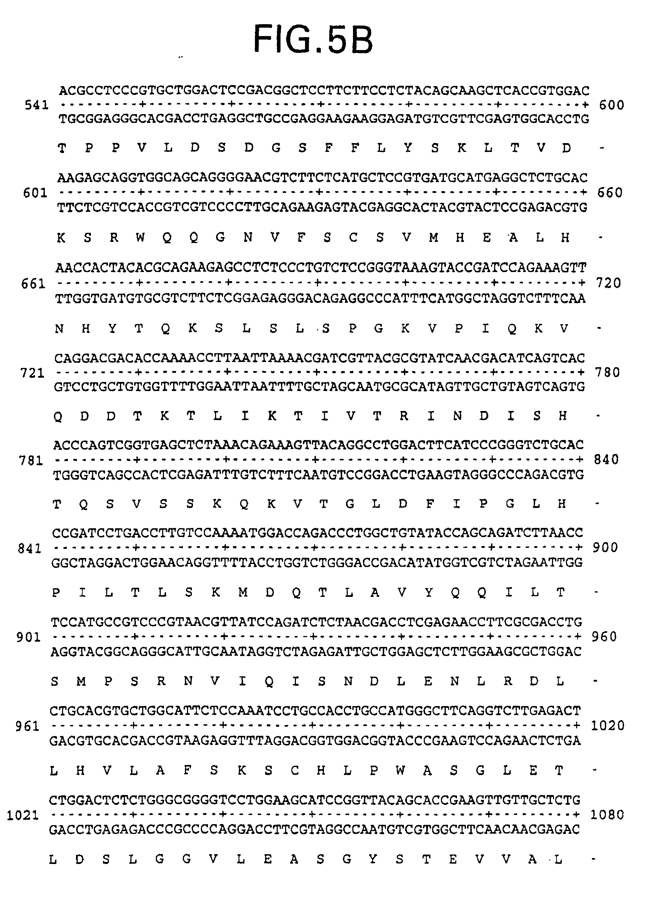

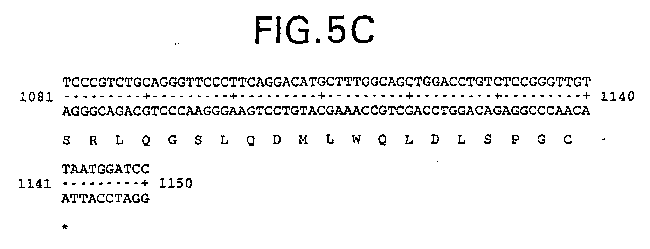

Preferably, an Fc region with the amino acid sequence of the human immunoglobulin IgG-1 heavy chain (see Ellison, J.W. *et al*., Nucleic Acids Res. 10: 4071-4079 (1982)) is used. A preferred Fc region is set forth in SEQ ID NO: 2 (see Figure 5). The recombinant Fc-OB sequence of SEQ ID NO: 2 is a 378 amino acid Fc-OB protein (not counting the methionine residue). The first amino acid residue of the Fc-OB protein in Figure 5, glutamic acid, is referred to as +1 with the methionine at the -1 position. Variants or analogs of the Fc portion may be constructed by, for example, making various substitutions of amino acid residues or base pairs.

Cysteine residues can be deleted or replaced with other amino acids to prevent formation of disulfide crosslinks of the Fc sequences. In particular, amino acid at position 5 of SEQ ID NO: 2 is a cysteine residue. One may remove the cysteine residue at position 5 or substitute it with one or more amino acids. For example, an alanine residue may be substituted for the cysteine residue at position 5 resulting in a variant amino acid sequence. Likewise, the cysteine at position 5 of SEQ ID NO: 2 may be substituted with a serine or other amino acid residue or deleted.

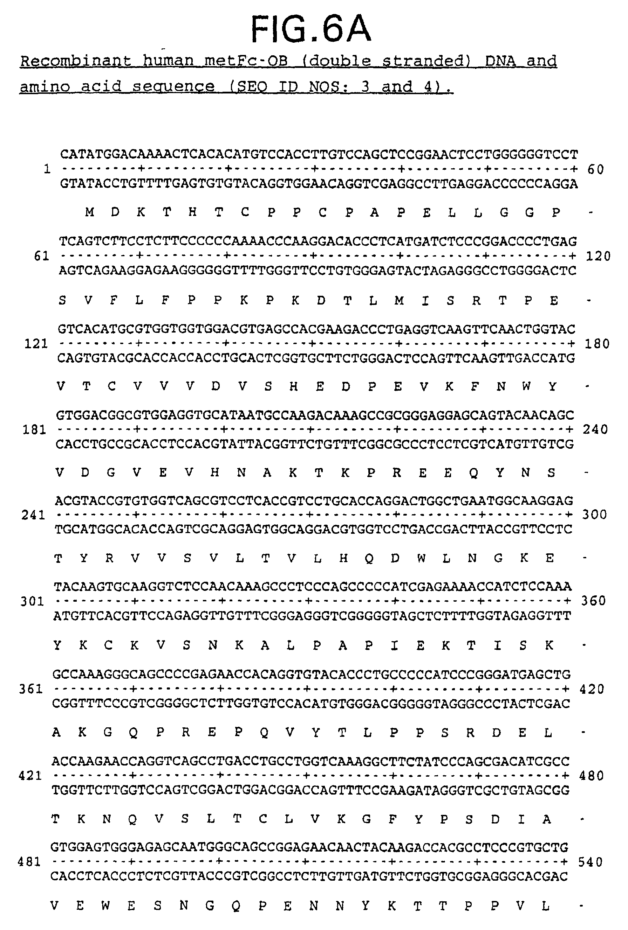

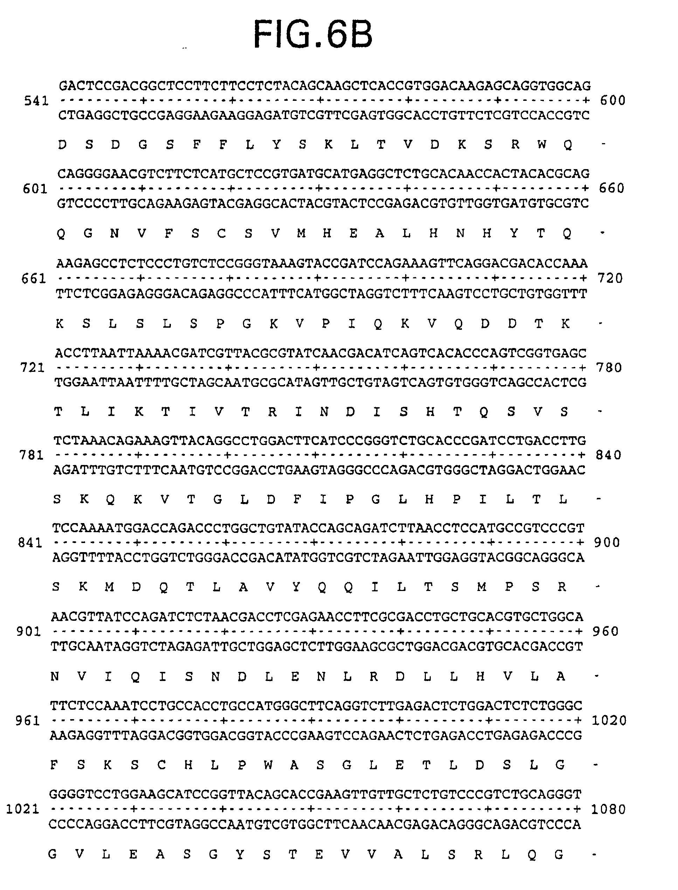

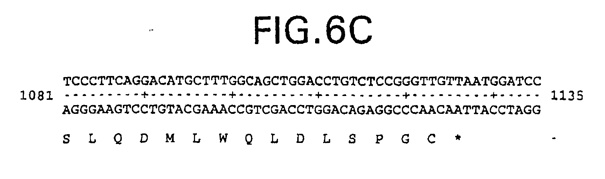

A variant or analog may also be prepared by deletion of the amino acids at positions 1, 2, 3, 4 and 5, resulting in a 373 amino acid Fc-OB protein (not counting the methionine residue). This sequence is set forth in SEQ ID NO:4 (see Figure 6). Substitutions at these positions can also be made and are within the scope of this invention.

Modifications may also be made to introduce four amino acid substitutions to ablate the Fc receptor binding site and the complement (C1q) binding site. According to the numbering of SEQ ID NO: 4, these variant modifications include leucine at position 15 substituted with glutamic acid, glutamic acid at position 98 substituted with alanine, and lysines at positions 100 and 102 substituted with alanines.

Likewise, one or more tyrosine residues can be replaced by phenylalanine residues as well. As described above, one may make changes in selected amino acids as long as such changes preserve the overall folding or activity of the fusion protein.

Furthermore, the Fc region may be also linked to the human OB protein of the Fc-OB fusion protein by "linker" moieties whether chemical or amino acids of varying lengths. Such chemical linkers are well known in the art. Amino acid linker sequences can include but are not limited to:
(a) ala, ala, ala;
(b) ala, ala, ala, ala;
(c) ala, ala, ala, ala, ala;
(d) gly, gly;
(e) gly, gly, gly;
(f) gly, gly, gly, gly, gly;
(g) gly, gly, gly, gly, gly, gly, gly;
(h) gly-pro-gly;
(i) gly, gly, pro, gly, gly; and
(j) any combination of subparts (a) through (i).

The present precipitating agents may be a salt having a cationic component, and may be selected from among calcium, magnesium, zinc, sodium, iron, cobalt, manganese, potassium and nickel. Preferably, the salt will be compatible for use in a pharmaceutical composition.

Alternatively precipitating agents may be selected from among agents which are pharmaceutically acceptable yet known to precipitate protein, such as polyethylene glycols, or other water soluble polymers as set forth in the next paragraphs. A useful precipitating agent will induce precipitation of OB protein at neutral pH but is reversible or redissolveable upon dilution with physiologically compatible solvents. Without an appropriate precipitating agent, OB protein precipitates at neutral pH to a form that is not reversible by dilution with physiologically compatible solvents.

The pH range is preferably from about pH 4.0 to about pH 8.0, and more preferably from about 6.5 to about 7.5. The most preferable pH for a pharmaceutical composition is that in which the OB protein used may retain its maximum biological activity at the selected protein concentration. At non-physiologic pH, the OB protein suspensions disclosed herein may also have advantages. At pH below 5.0, the OB protein suspensions may be more stable (in terms of shelf-life) than equal concentrations of OB protein in solution at equal pH. For example at pH 4.0, and a concentration of 50 mg/ml, the suspensions may have greater biological activity upon in vivo administration than would the equivalent solution.

The buffer may be selected from among those which attain the desired pH while not altering the precipitating characteristics of the composition. Preferably, buffers will be acceptable for a pharmaceutical formulation. Tris, MES, and PIPES are acceptable for both the amorphous and crystalline forms. Phosphate is a preferred buffer for the crystalline forms.

The final suspension will preferably have a concentration of 5 mg/ml to 100 mg/ml for ease of therapeutic administration.

### Msthods of Use

Therapeutic. Therapeutic uses include weight modulation, the treatment or prevention *of* diabetes, blood lipid reduction (and treatment of related conditions), increasing lean body mass and increasing insulin sensitivity. In addition, the present suspensions may be used for manufacture of one or more medicaments for treatment or amelioration of the above conditions. Methods of administration will typically be by injection, although other means, such as pulmonary delivery may be used. See PCT WO 96/05309, at page 83 et seq., for example. The present suspensions may be spray-dried into particles having an average size of less than 10 microns, or more preferably, 0.5 to 5 microns.

Weight Modulation. The present suspensions and methods may be used for weight reduction. Viewed another way, the present suspensions may be used for maintenance of a desired weight or level of adiposity. As has been demonstrated in murine models (see supra), administration of OB protein results in weight loss. The body mass lost is primarily of adipose tissue, or fat. such weight loss can be associated with the treatment of concomitant conditions, such as those below, and therefore constitute a therapeutic application. In addition, cosmetic uses are provided herein if weight modulation is solely for improvement in appearance.

Treatment of Diabetes. The present suspensions and methods may be used in the prevention or treatment of Type II diabetes. As Type II diabetes can be correlated with obesity, use of the present invention to reduce weight (or maintain a desired weight, or reduce or maintain an adiposity level) can also alleviate or prevent the development of diabetes. Moreover, even in the absence of dosages sufficient to result in weight loss, the present suspensions may be used to prevent or ameliorate diabetes.

Blood Lipid Modulation. The present suspensions and methods may be used in the modulation of blood lipid levels. Ideally, in situations where solely reduction in blood lipid levels is desired, or where maintenance of blood lipid levels is desired, the dosage will be insufficient to result in weight loss. Thus, during an initial course of therapy of an obese patient, dosages may be administered whereby weight loss and concomitant blood lipid level lowering is achieved. Once sufficient weight loss is achieved, a dosage sufficient to prevent re-gaining weight, yet sufficient to maintain desired blood lipid levels, or other conditions as set forth herein, for example, may be administered. These dosages can be determined empirically, as the effects of OB protein are reversible. E.g., Campfield et al., Science 269: 546-549 (1995) at 547. Thus, if a dosage resulting in weight loss is observed when weight loss is not desired, one would administer a lower dose in order to achieve the desired blood lipid levels, yet maintain the desired weight. See, e.g., PCT Publication WO 97/06816.

### Increasing Lean Mass or Insulin Sensitivity.

Ideally, in situations where solely an increase in lean body mass is desired, the dosage will be insufficient to result in weight loss. Thus, during an initial course of therapy of an obese person, dosages may be administered whereby weight loss and concomitant fat tissue decrease/lean mass increase is achieved. Once sufficient weight loss is achieved, a dosage sufficient to prevent regaining weight, yet sufficient to maintain desired lean mass increase (or prevention of lean mass depletion) may be administered. For increasing an individual's sensitivity to insulin, similar dosage considerations may be taken into account. Lean mass increase without weight loss may be achieved sufficient to decrease the amount of insulin (or, potentially, amylin, amylin antagonists or agonists, or thiazolidinediones, or other potential diabetes treating drugs) an individual would be administered for the treatment of diabetes. For increasing overall strength, there may be similar dosage considerations. Lean mass increase with concomitant increase in overall strength may be achieved with doses insufficient to result in weight loss. Other benefits, such as an increase in red blood cells (and oxygenation in the blood) and a decrease in bone resorption or osteoporosis may also be achieved in the absence of weight loss. See, e.g., PCT Publication No. WO 97/18833.

Combination Therapies. The present suspensions and methods may be used in conjunction with other therapies, such as altered diet and exercise. Other medicaments, such as those useful for the treatment of diabetes (e.g., insulin and possibly amylin, antagonists or agonists thereof, thiazolidinediones (see, e.g., PCT Publication No. WO 98/08512), or other potential diabetes treating drugs), cholesterol and blood pressure lowering medicaments (such as those which reduce blood lipid levels or other cardiovascular medicaments), activity increasing medicaments (e.g., amphetamines), diuretics (for liquid elimination), and appetite suppressants (such as agents which act on neuropeptide γ receptors or serotonin reuptake inhibitors). Such administration may be simultaneous or may be in seriatim. In addition, the suspensions of the present invention may be used in conjunction with surgical procedures, such as cosmetic surgeries designed to alter the overall appearance of a body (e.g., liposuction or laser surgeries designed to reduce body mass, or implant surgeries designed to increase the appearance of body mass). The health benefits of cardiac surgeries, such as bypass surgeries or other surgeries designed to relieve a deleterious condition caused by blockage of blood vessels by fatty deposits, such as arterial plaque, may be increased with concomitant use of the present suspensions and methods. Methods to eliminate gall stones, such as ultrasonic or laser. methods, may also be used either prior to during or after a course of the therapeutic methods disclosed herein. Furthermore, the suspensions of the present invention may be used as an adjunct to surgeries or therapies for broken bones, damaged muscle, or other therapies which would be improved by an increase in lean tissue mass.

The following examples are offered to more fully illustrate the invention, but are not to be construed as limiting the scope thereof. Example 1 sets forth preparation of an amorphous (as opposed to a crystalline, as infra) human OB protein suspension at a concentration of 100 mg/ml at pH 7.0. Example 2 sets forth preparation of a crystalline OB protein suspension. Example 3 demonstrates an improved dose response for OB protein suspensions as compared to OB protein solutions. Example 4 demonstrates the delayed time action profile of OB protein suspensions in a dog model. Example 5 sets forth preparation of an amorphous human Fc-OB protein suspension according to the present invention. Examples 6 and 7 demonstrate the improved stability of the suspensions.

### EXAMPLE 1: Preparation of an amorphous OB protein suspension.

This example illustrates preparation of a human OB protein suspension (not according to the claimed invention). An amorphous human OB protein suspension was prepared by precipitation with zinc salt. At a final pH of 6.0 to pH 8.0, a concentration of 100 mg protein/ml liquid has been obtained. Also set forth is a control composition, of pH 4.0, for'a human OB protein solution.

### Composition:

Protein moiety: recombinant methionyl human OB protein ("rmetHu-leptin") as set forth in SEQ ID NO. 4 of PCT Publication WO 96/05309, beginning with amino acid number 22 (Val) and ending with amino acid number 167, having at its N-terminus a methionyl residue:
Precipitant: Zinc chloride
Buffer: Tris, MES and Pipes
Final pH; 6.0-8.0
Preparation protocol: Recombinant methionyl human OB protein ("rmetHu-leptin") solution was concentrated to about 40 mg/ml in water for injection, acidified to pH 3.0, with HCl. Zinc chloride was added and the suspension was formed by adjusting the pH to near neutrality (approximately pH 7.0) by adding an appropriate buffer. Tris, MES and PIPES buffer have been successfully used. The final conditions were typically 10 to 15 mM buffer, 20 to 1000 µM Zinc, pH 6.0 to 8.0 and 10 mg/ml rmetHu-leptin. The suspensions have been concentrated by allowing the particles to settle at 4°C for several hours and removing the supernatant. This procedure may be repeated several times until the maximum concentration is obtained, and has been used to obtain about 100 mg/ml suspensions.
Control Composition: Recombinant methionyl human OB protein (as above) solution at 20 mg/ml pH 4.0 containing 10 mM acetate and 5% w/v sorbitol was used as a control composition.

### EXAMPLE 2: Preparation of a crystalline OB protein suspension.

This example illustrates the preparation of a crystalline OB protein suspension (not according to the claimed invention).

Protein moiety: recombinant methionyl human OB protein as in Example 1, above, was used.

Procedure: rmetHu-leptin at a concentration of 15 mg/ml in 1 mM HCl was mixed in a 1:1 ratio with 4 M NaCl, 100 mM Tris, pH 8.5, 2% v/v ethanol, at 4°C. Crystals spontaneously formed by slowly adjusting the temperature over several hours to between 14°C and 25°C and maintaining that temperature for at least 2 hours depending on the duration of warming to the final temperature. The "mother liquor" (i.e., the liquid in which the crystals were grown) was replaced with a more suitable solvent for injection by harvesting the crystals by centrifugation and resuspension in an appropriate crystal stabilizing solvent. A suitable replacement solvent is 20-25% polyethylene glycol (having a molecular weight of about 4000 daltons to about 20,000 daltons, the term "about" meaning that the approximate average of molecular weights for commercial PEG preparations), appropriate neutral pH buffer, preferably about pH 6.0 to about pH 8.0, and preferably 10 mM phosphate buffer pH 6.0 to pH 7.5, and 2% ethanol v/v. In a typical preparation, some residual salt, usually less than 0.25 M, may remain.

### EXAMPLE 3: Improved dose response for OB protein suspensions as compared to OB protein solutions.

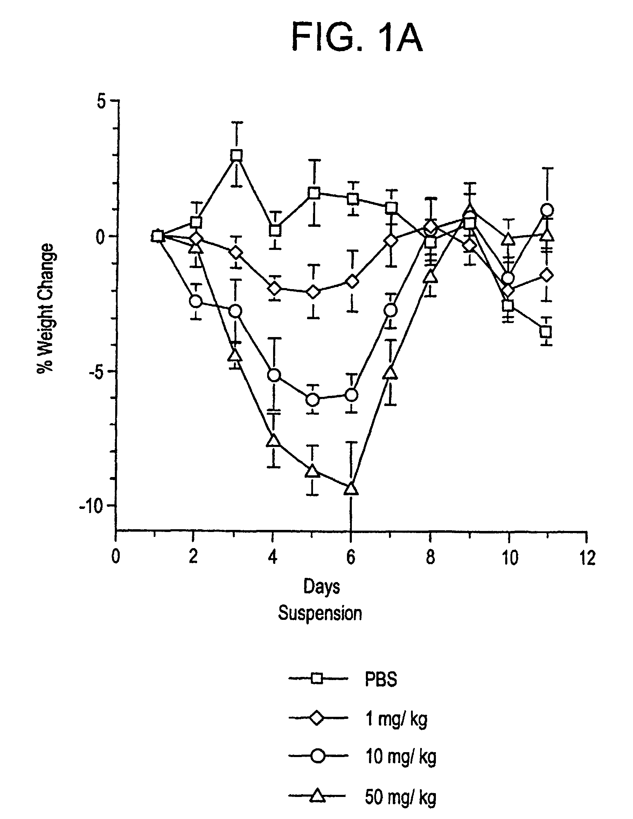

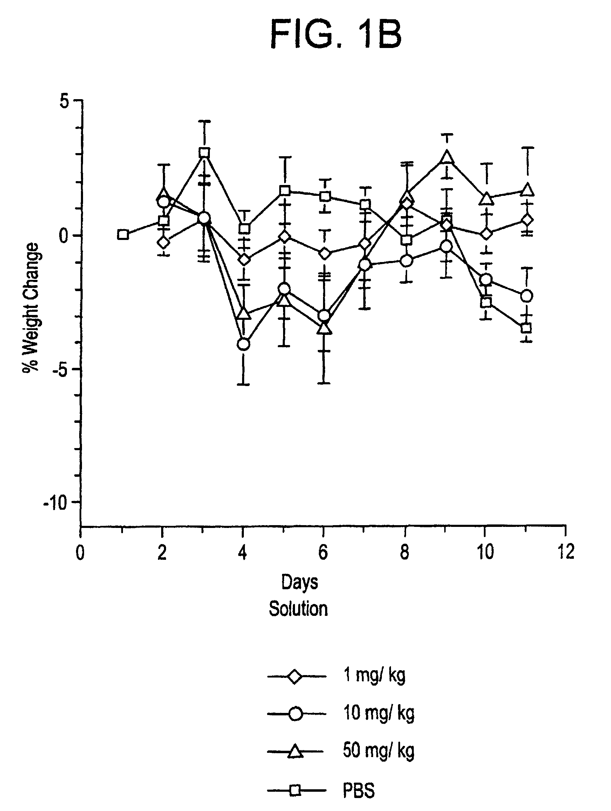

This example demonstrates that OB protein suspensions are more efficacious than OB protein in solution. Normal lean mice were given daily injections, for 5 days, of either the suspension, at 1, 10 and 50 mg of protein per kg of body weight, or OB protein solution at the same dose. The mice given the suspension lost more weight per unit of mass of OB protein given than did mice given equal doses of the solution formulation. This is illustrated in FIGURE 1A and FIGURE 1B. FIGURE 1A shows the percent weight change when the suspension of Example 1 was given. FIGURE 1B shows the percent weight change when the control solution of Example 1 was given.

This illustrates that the suspension at the same dose is more efficacious than that given in solution form. While not wishing to be bound by theory, this may be due to slower absorption rate of the suspension as compared to the solution. The suspension must dissolve before it enters the blood, so this sustained release effect results in higher efficacy. On a mass basis, less protein in suspension needs to be administered than solution. Further, in solution there is no difference between the 10 and 50 mg/kg dose at day 5 (the last day of dosing) (See Table 2, below). The suspension gives a more definitive dose response curve than does the solution formulation.

**TABLE 2**

| PERCENT WEIGHT CHANGE FROM DAY 0 | |
|---|---|
| Day | 6 |
| Placebo | 1.4 |
| Suspension 1 mg/kg | -1.7 |
| Suspension 10 mg/kg | -5.9 |
| Suspension 50 mg/kg | -9.5 |
| Solution 1 mg/kg | -.7 |
| Solution 10 mg/kg | -3.0 |
| Solution 50 mg/kg | -3.5 |
| The date is an average for 5 mice per treatment. | |

### Methods:

Animals: Normal CD-1 mice were used.
Base weight: About 20 grams.
Administration: Animals were injected SC each day in the same location for 5 days.
Handling: Animals were group housed, and fed ad libitum.

### Compositions:

Solution: The rmetHu-leptin solution of Example 1 was used, at a concentration of 20 mg/ml.
Suspension: The rmetHu-leptin suspension of Example 1 was used, at pH 7.0, 10 mM MES buffer, 500 mM Zn, 20 mg/ml.
PBS: Phosphate buffered saline was used as a placebo. (Dose responses for controls using suspension liquid or solution liquid alone, with no protein, were similar to PBS, data not shown).

### EXAMPLE 4: OB protein serum levels in dogs.

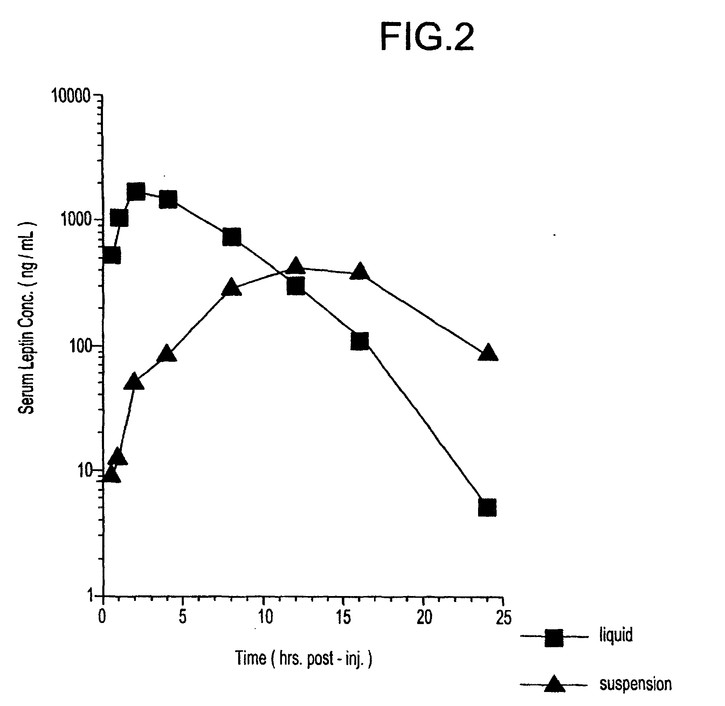

This Example demonstrates the delayed time action profile of the suspensions of Example 2. Methods: Beagle dogs were administered suspension or solution rmetHu-leptin. Serum was drawn, and OB protein levels were measured at the times illustrated in FIGURE 2.

**TABLE 3**

| Time after injection (hours) | Serum concentration Solution | Serum concentration Suspension (crystal) | Serum concentration Suspension (zinc) |
|---|---|---|---|
| 0 | 0 | 0 | 0 |
| 5 | 532 | 9.4 | 50.5 |
| 1 | 1047 | 12.9 | 130.4 |
| 2 | 1706 | 51 | 298.5 |
| 4 | 1480 | 87 | 323.5 |
| 8 | 742 | 298 | 247.7 |
| 12 | 301 | 426 | 241.1 |
| 16 | 109 | 389 | 209.6 |
| 24 | 5 | 90 | 57.7 |

### Compositions used:

Solution: The solution according to Example 1 was used, at a dose of 5 mg/kg/day.
Suspension: The suspension according to Example 2 was used. See Table.
Animals: The data presented in Table 3 is for an average of 3 animals. Animals were normal beagle dogs.
Handling: Animals were housed individually, and fed ad libitum. Good animal handling practices were obeyed.
Assay: An antibody assay was used as in Hotta et al, J. Biol. Chem 271: 255327-25331 (1996).
Results: As can be seen from FIGURE 2, the solution concentration peaks at 1 to 2 hours after administration of protein, whereas the suspension concentration peaks much later, after 10-12 hours. This demonstrates that the suspension maintains a minimum effective dose for a longer time period.

### EXAMPLE 5: Preparation of an amorphous Fc-OB protein suspension.

This example illustrates preparation of one of the human Fc-OB protein suspensions of the present invention. An amorphous human Fc-OB protein suspension was prepared by precipitation with zinc salt. Also set forth is a control composition, of pH 7.5, for a human Fc-OB protein solution.

### Composition:

Protein moiety: recombinant methionyl human Fc-OB protein ("rmetHu-Fc-leptin") as set forth in SEQ ID NO: 4, a 373 amino acid fusion protein, the Fc portion of the protein being amino acids 1-227 and the human OB protein portion of the protein being amino acids 228-373, having at its N-terminus a methionyl residue:
Precipitant: Zinc chloride
Buffer: 100mM Tris
Final pH: 7.5
Preparation protocol: A rmetHu-Fc-leptin solution was concentrated to about 5 mg/ml in 100 mM Tris buffer, pH 7.5. Zinc chloride was added to form the suspension.
Control Composition: A rmetHu-Fc-leptin solution (as above) at 5 mg/ml, pH 7.5 containing 100 mM Tris was used as a control composition.

### EXAMPLE 6: Stability of human OB protein suspensions.

This example demonstrates that both the amorphous and the crystalline forms of the suspensions of Examples 1 and 2, respectively, are more stable under accelerated stability assay conditions than material in solution.

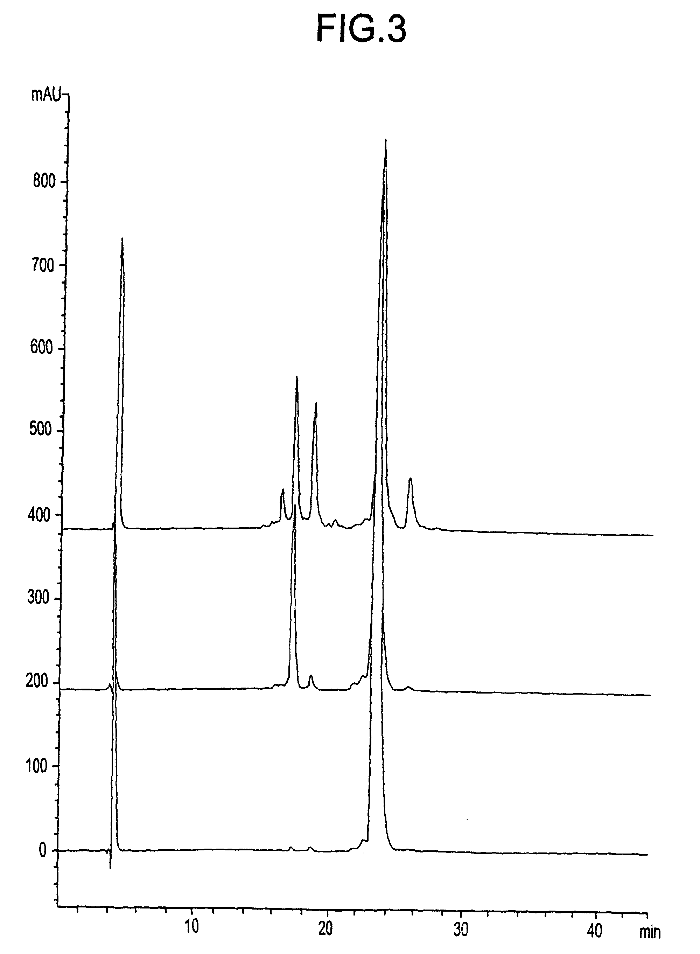

FIGURE 3 illustrates an RP-HPLC tracing comparing the zinc amorphous form of the suspension (as in Example 1) with the solution form (also as in Example 1), at 37°C for a period of 7 weeks. As can be seen, the suspension (middle line) has fewer peaks (indicating fewer breakdown products) than the solution form (top line). As a comparison, the bottom line presents the zinc amorphous form which had been stored for 7 weeks at -80°C.

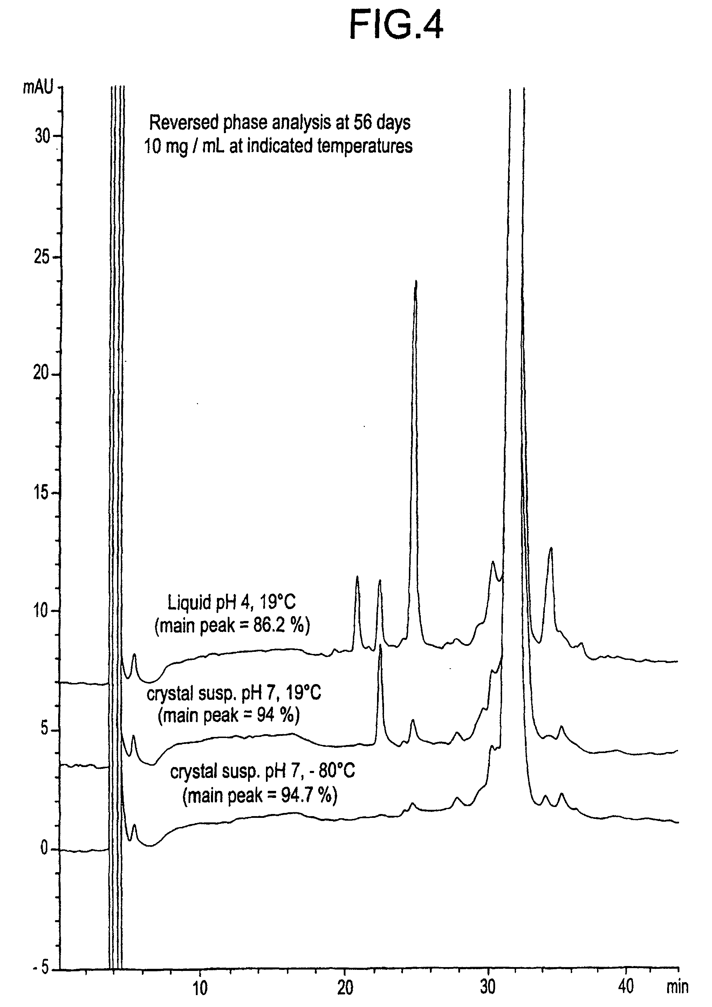

FIGURE 4 illustrates an RP-HPLC tracing comparing the crystalline suspension form of Example 2 with the solution form (of Example 1). The materials were stored for 56 days at 19°C. (Storage at 37°C was not a relevant condition, as the crystalline form loses its crystalline structure at 37°C.) FIGURE 4 shows that there are more peaks, indicating more degradation, for the solution form, (main peak = 86%) than for the suspension form (main peak = 94%). The control at the bottom line was the crystalline form stored for 56 days at -80°C. Similar results were observed for 4°C, although degradation occurred more slowly.

The main degradation product appeared to be the aspartate at amino acid position 108 (according to SEQ ID NO: 4 in PCT WO 96/05309, using the "Val" residue as position number 1). One may empirically select a more stable chemical moiety at this position, such as another amino acid, to improve stability of the molecule. Overall, the suspensions are more stable than OB protein in solution.

### EXAMPLE 7: Stability of the human Fc-OB protein suspensions of the present invention.

This example demonstrates that the amorphous form of the present Fc-OB protein suspensions is more stable under accelerated stability assay conditions than material in solution.

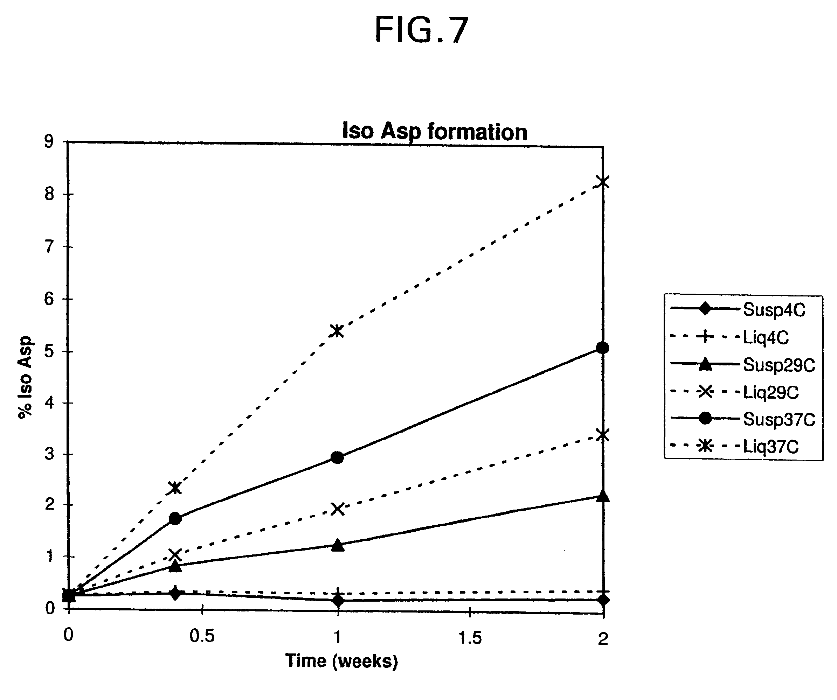

The zinc amorphous form of the present suspension (as in Example 5) and the solution form (also as in Example 5) were put on stability studies by storing both the suspension and solution forms at 4°C, 29°C and 37°C for a period of 2 weeks. As shown in FIGURE 7, the Fc-leptin suspensions stored at 29°C and 37°C show a lower percentage of degradation products related to the aspartate at amino acid position 108 of the OB protein than the Fc-leptin solutions stored at the same temperatures, respectively.

While the present invention has been described in terms of preferred embodiments, it is understood that variations and modifications will occur to those skilled in the art. Therefore, it is intended that the appended claims cover all such equivalent variations which come within the scope of the invention as claimed.

## Claims

1. A suspension having a pH of 6.0 to 8.0 of human OB protein derivatized by the attachment of an Fc region of an immunoglabulin to the N-terminus of the OB protein moiety at a concentration of at least 0.5 mg/ml, said suspension also containing a precipitating agent.

2. A suspension of claim 1 wherein the Fc portion of said human Fc-OB protein is selected from the group consisting of;
(a) the Fc amino acid sequences as set forth in SEQ ID NOS: 2 and 4;
(b) the amino acid sequence of subpart (a) having a different amino acid substituted or deleted in one or more of the following positions (using the numbering according to SEQ ID NO: 2):
(i) one or more cysteine residues replaced by an alanine or serine residue;
(ii) one or more tyrosine residues replaced by a phenylalanine residue;
(iii) the amino acid at position 5 replaced with an alanine;
(iv) the amino acid at position 20 replaced with glutamic acid;
(v) the amino acid at position 103 replaced with an alanine;
(vi) the amino acid at position 105 replaced with an alanine;
(vii) the amino acid at position 107 replaced with an alanine;
(viii) the amino acids at positions 1, 2, 3, 4, and 5 deleted;
(ix) one or more residues replaced or deleted to ablate the Fc receptor binding site;
(x) one or more residues substituted or deleted to ablate the complement (C1q) binding site; and
(xi) a combination of subparts i-x; and
(c) the amino acid sequence of subparts (a) or (b) having a methionyl residue at the N-terminus.

3. A suspension of claim 1 wherein said concentration is at least 5 mg/ml.

4. A suspension of claim 1 wherein said concentration is between 5 mg/ml and 50 mg/ml.

5. A suspension of claim 1 wherein said pH is pH 7.0.

6. A suspension of claim 2 wherein said human OB protein is rmetHu-Fc-leptin.

7. A suspension of claim 1 containing a precipitating agent selected from a salt and a water soluble polymer.

8. A suspension of claim 1 containing a precipitating agent selected from among calcium, magnesium, zinc, sodium, iron, cobalt, manganese, potassium and nickel.

9. A method of preparing a suspension of any one of claims 1 to 8, comprising:
combining, under suitable conditions, a precipitating agent and a human OB protein in solution, wherein the OB protein is derivatized by the attachment of an Fc region of an immunoglobulin to the N-terminus of the OB protein moiety; allowing said human OB protein to precipitate;
collecting said precipitated human OB protein; and
resuspending said human OB protein in a diluent.

10. A suspension according to any one of Claims 1 to 8 for therapeutic administration.

11. Use of a suspension according to any one of Claims 1 to 8 for the manufacture of a medicament for treating a condition selected from:
(a) weight modulation,
(b) adiposity modulation,
(c) diabetes,
(d) blood lipid level modulation,
(e) increase in lean mass, and
(f) increase in insulin sensitivity.

12. A pharmaceutical composition comprising a suspension according to any one of Claims 1 to 8.

13. A method of cosmetically treating an individual comprising administering to said individual an effective dose of a suspension according to any one of Claims 1 to 8.

## Patentansprüche

1. Suspension mit einem pH von 6,0 bis 8,0 von menschlichem OB-Protein, das durch Anhängen einer Fc-Region eines Immunglobulins an den N-Terminus der OB-Proteingruppe derivatisiert ist, in einer Konzentration von wenigstens 0,5 mg/ml, wobei besagte Suspension ebenso ein Fällungsmittel enthält.

2. Suspension nach Anspruch 1, wobei der Fc-Teil von besagtem menschlichen Fc-OB-Protein ausgewählt ist aus der Gruppe, bestehend aus:
(a) den Fc-Aminosäuresequenzen, wie dargestellt in SEQ ID NO: 2 und 4;
(b) der Aminosäuresequenz eines Teils (a) mit einer unterschiedlichen Aminosäure, die an einer oder mehrerer der folgenden Positionen substituiert oder deletiert ist (unter Verwendung der Zählung gemäß SEQ ID NO: 2):
(i) ein oder mehrere Cysteinreste, ersetzt durch einen Alanin- oder Serinrest;
(ii) ein oder mehrere Tyrosinreste, ersetzt durch einen Phenylalaninrest;
(iii) die Aminosäure an Position 5, ersetzt durch ein Alanin;
(iv) die Aminosäure an Position 20, ersetzt durch eine Glutaminsäure;
(v) die Aminosäure an Position 103, ersetzt durch ein Alanin;
(vi) die Aminosäure an Position 105, ersetzt durch ein Alanin;
(vii) die Aminosäure an Position 107, ersetzt durch ein Alanin;
(viii) die Aminosäuren an Positionen 1, 2, 3, 4 und 5 deletiert;
(ix) ein oder mehrere Reste ersetzt oder deletiert, um die Fc-Rezeptor-Bindungsstelle zu entfernen;
(x) ein oder mehrere Reste substituiert oder deletiert, um die Komplement-(C1q)-Bindungsstelle zu entfernen; und
(xi) eine Kombination der Teile i-x; und
(c) der Aminosäuresequenz der Teile (a) oder (b) mit einem Methionylrest am N-Terminus.

3. Suspension nach Anspruch 1, wobei besagte Konzentration wenigstens 5 mg/ml beträgt.

4. Suspension nach Anspruch 1, wobei besagte Konzentration zwischen 5 mg/ml und 50 mg/ml beträgt.

5. Suspension nach Anspruch 1, wobei besagter pH pH 7,0 ist.

6. Suspension nach Anspruch 2, wobei besagtes menschliches OB-Protein rmetHu-Fc-Leptin ist.

7. Suspension nach Anspruch 1, enthaltend ein Fällungsmittel, ausgewählt aus einem Salz und einem wasserlöslichen Polymer.

8. Suspension nach Anspruch 1, enthaltend ein Fällungsmittel, ausgewählt aus Calcium, Magnesium, Zink, Natrium, Eisen, Kobalt, Mangan, Kalium und Nickel.

9. Verfahren zum Herstellung einer Suspension nach einem der Ansprüche 1 bis 8, umfassend:
Kombinieren, unter geeigneten Bedingungen, eines Fällungsmittels und eines menschlichen OB-Proteins in Lösung, wobei das OB-Protein durch das Anhängen einer Fc-Region eines Immunglobulins an den N-Terminus der OB-Proteingruppe derivatisiert ist, Ausfällen von besagtem menschlichem OB-Protein;
Sammeln von besagtem ausgefälltem, menschlichen OB-Protein; und
Resuspendieren von besagtem menschlichen OB-Protein in einem Verdünnungsmittel.

10. Suspension nach einem der Ansprüche 1 bis 8 zur therapeutischen Verabreichung.

11. Verwendung einer Suspension nach einem der Ansprüche 1 bis 8 zur Herstellung eines Medikaments zur Behandlung eines Zustands, ausgewählt aus:
(a) Modulierung des Gewichts,
(b) Modulierung der Fettleibigkeit,
(c) Diabetes,
(d) Modulierung der Blutlipidkonzentration,
(e) Erhöhung der Magermasse (lean mass), und
(f) Erhöhung der Insulinempfindlichkeit.

12. Pharmazeutische Zusammensetzung, umfassend eine Suspension nach einem der Ansprüche 1 bis 8.

13. Verfahren zur kosmetischen Behandlung eines Individuums, umfassend die Verabreichung an besagtes Individuum einer wirksamen Dosis einer Suspension nach einem der Ansprüche 1 bis 8.

## Revendications

1. Suspension ayant un pH de 6,0 à 8,0 de protéine OB humaine dérivée par la fixation d'une région Fc d'une immunoglobuline à la terminaison N de la fraction de protéine OB à une concentration d'au moins 0,5 mg/ml, ladite suspension contenant également un agent précipitant.

2. Suspension de la revendication 1, dans laquelle la portion Fc de ladite protéine Fc-OB humaine est choisie dans le groupe constitué de :
(a) les séquences d'acides aminés Fc comme indiqué dans SEQ ID NOS : 2 et 4 ;
(b) la séquence d'acides aminés de la sous-partie (a) ayant un acide aminé différent substitué ou supprimé dans une ou plusieurs des positions suivantes (en utilisant la numérotation selon SEQ ID NO : 2) :
(i) un ou plusieurs résidus de cystéine remplacés par un résidu d'alanine ou de sérine ;
(ii) un ou plusieurs résidus de tyrosine remplacés par un résidu de phénylalanine ;
(iii) l'acide aminé en position 5 remplacé par une alanine ;
(iv) l'acide aminé en position 20 remplacé par de l'acide glutamique ;
(v) l'acide aminé en position 103 remplacé par une alanine ;
(vi) l'acide aminé en position 105 remplacé par une alanine ;
(vii) l'acide aminé en position 107 remplacé par une alanine ;
(viii) les acides aminés en positions 1, 2, 3, 4, et 5 supprimés ;
(ix) un ou plusieurs résidus remplacés ou supprimés pour pratiquer l'ablation du site de liaison du récepteur Fc ;
(x) un ou plusieurs résidus substitués ou supprimés pour pratiquer l'ablation du site de liaison de complément (C1q) ; et
(xi) une combinaison des sous-parties i-x ; et
(c) la séquence d'acides aminés de la sous-partie (a) ou (b) ayant un résidu de méthionyle à la terminaison N.

3. Suspension de la revendication 1, dans laquelle ladite concentration est au moins de 5 mg/ml.

4. Suspension de la revendication 1, dans laquelle ladite concentration est entre 5 mg/ml et 50 mg/ml.

5. Suspension de la revendication 1, dans laquelle ledit pH est pH 7,0.

6. Suspension de la revendication 2, dans laquelle ladite protéine OB humaine est la rmetHu-Fc-leptine.

7. Suspension de la revendication 1, contenant un agent précipitant choisi à partir d'un sel et d'un polymère soluble dans l'eau.

8. Suspension de la revendication 1, contenant un agent précipitant choisi parmi le calcium, le magnésium, le zinc, le sodium, le fer, le cobalt, le manganèse, le potassium et le nickel.

9. Procédé pour préparer une suspension selon une quelconque des revendications 1 à 8, comprenant :
- combiner, dans des conditions appropriées, un agent précipitant et une protéine OB humaine en solution, où la protéine OB est dérivée par la fixation d'une région Fc d'une immunoglobuline à la terminaison N de la fraction de protéine OB, permettant à ladite protéine OB humaine de précipiter ;
- recueillir ladite protéine OB humaine précipitée ; et
- resuspendre ladite protéine OB humaine dans un diluant.

10. Suspension selon une quelconque des revendications 1 à 8 pour une administration thérapeutique.

11. Utilisation d'une suspension selon une quelconque des revendications 1 à 8 pour la fabrication d'un médicament pour traiter une condition choisie parmi :
(a) modulation du poids,
(b) modulation de l'adiposité,
(c) diabète,
(d) modulation du taux de lipides dans le sang
(e) augmentation de la masse maigre, et
(f) augmentation de la sensibilité à l'insuline.

12. Composition pharmaceutique comprenant une suspension selon une quelconque des revendications 1 à 8.

13. Procédé de traitement cosmétique d'un individu comprenant l'administration audit individu d'une dose efficace d'une suspension selon une quelconque des revendications 1 à 8.
